# EUROPÄISCHE PATENTSCHRIFT

(11) **EP 2 367 780 B1**
(45) Veröffentlichungstag und Bekanntmachung des Hinweises auf die Patenterteilung: **19.11.2014**
(21) Anmeldenummer: 09775097.0
(22) Anmeldetag: 19.11.2009
(51) Int. Cl.: C07C 41/09, C07C 43/04, B01D 3/14

(54) **VERFAHREN UND VORRICHTUNG ZUM HERSTELLEN VON DIMETHYLETHER AUS METHANOL**
METHOD AND DEVICE FOR PRODUCING DIMETHYL ETHER FROM METHANOL
PROCÉDÉ ET DISPOSITIF DE PRODUCTION DE DIMÉTHYLÉTHER À PARTIR DE MÉTHANOL

(30) Priorität: 25.11.2008 DE 102008058931
(43) Veröffentlichungstag der Anmeldung: 28.09.2011
(73) Patentinhaber: Air Liquide Global E&C Solutions Germany GmbH, 60439 Frankfurt am Main (DE)
(72) Erfinder: AHLERS, Bernd, 63128 Dietzenbach (DE); BIRKE, Gerhard, 60389 Frankfurt a. M. (DE); KÖMPEL, Harald, 63263 Neu-Isenburg (DE); BACH, Hermann, 56412 Heiligenroth (DE); ROTHÄMEL, Martin, 60437 Frankfurt am Main (DE); LIEBNER, Waldemar, 61440 Oberursel (DE); BOII, Walter, 60388 Frankfurt am Main (DE); GRONEMANN, Veronika, 61184 Karben (DE)
(86) Internationale Anmeldenummer: PCT/EP2009/008237
(87) Internationale Veröffentlichungsnummer: WO 2010/060566

(56) Entgegenhaltungen:
- EP-A1- 0 455 004
- WO-A1-2008/026887
- US-A- 5 750 799

## Beschreibung

Die Erfindung betrifft ein Verfahren und eine Vorrichtung zum Herstellen von Dimethylether (DME) aus Methanol (MeOH) durch Umsetzen, vorzugsweise durch säurenkatalytische Kondensation, von durch MeOH-Synthese gewonnenem Roh-MeOH zu DME unter Abspalten von Wasser in einem Reaktor, bei dem das aus Roh-MeOH bestehende Einsatzgemisch und ein prozessintern gewonnener im wesentlichen aus nicht umgesetztem MeOH und Reaktionswasser gebildeter Rückfluss einer Kolonne, nachfolgend als MeOH-Kolonne bezeichnet, aufgegeben und verdampft werden und das im wesentlichen aus dampfförmigem MeOH bestehende Destillat dem Reaktor zugeführt wird.

MeOH wird heute ausschließlich aus den Synthesegasen CO₂/H₂ oder CO/H₂ hergestellt, die ihrerseits aus der Reformierung von Erdgas, Rückstandsölen der Erdölaufbereitung oder aus der Druckvergasung von Kohle stammen. Das erzeugte Roh-MeOH kann unmittelbar zu DME weiterverarbeitet oder destillativ zu Rein-MeOH aufgearbeitet und anschließend katalytisch zu DME und Wasser umgesetzt werden. In beiden Fällen wird das gewonnene DME-Produkt destillativ von nicht umgesetztem MeOH und Wasser getrennt. In aller Regel wird das Roh-MeOH einer zwei- oder dreistufigen Destillation, in der zunächst die Leichtsieder und die gelösten Gase, insbesondere CO₂, entfernt und dann MeOH und Wasser getrennt werden, unterworfen und das gereinigte MeOH in einem adiabaten Festbettreaktor bis zum Reaktionsgleichgewicht zu DME umgesetzt. Da das Reaktorprodukt aus einem Gemisch von DME, Wasser, nicht-umgesetztem MeOH und kleineren Mengen nichtkondensierbarer leichter Gase besteht, wird das Reaktorprodukt in einer zweistufigen Destillation behandelt, wobei in der ersten Destillationsstufe im Kopf DME von nicht-umgesetztem MeOH und Reaktionswasser und in der zweiten Destillationsstufe im Kopf das in dem Sumpfprodukt der ersten Stufe enthaltene MeOH von dem Reaktionswasser getrennt werden und das gewonnene MeOH zurück in den Reaktor fließt. Die am Kopf der ersten Destillationsstufe mit dem DME ausgetragenen nicht-kondensierbaren leichten Gase sind mit DME gesättigt, das in einer Gaswaschstufe mit MeOH als Waschmittel von den nicht-kondensierbaren Gasen getrennt wird, bevor diese als Leichtsieder das System am Kopf die Gaswaschstufe verlassen. Es werden demnach sowohl bei der Destillation von DME als auch bei der Destillation von MeOH, vorwiegend aus MeOH, Wasser und DME bestehende Stoffgemische, getrennt. Da die Produktspezifikationen für DME einerseits und MeOH andererseits verschiedenen Erfordernissen genügen müssen, werden die Destillationen von DME und MeOH separat durchgeführt. Die vorstehend beschriebenen Maßnahmen werden insbesondere für das Herstellen von hochreinem DME, der breite Anwendung als Treibgas, z.B. in Haarspray und Lackspray findet, angewendet. Technischer DME ist eine Alternative zu Flüssiggasen mit ausgezeichneten Brenneigenschaften. Aufgrund einer Cetanzahl von 55 bis 60 lässt sich DME in Dieselmotoren als Ersatz für Dieselkraftstoff einsetzen.

Da gemäß der Biokraftstoffrichtlinie 2003/30/EG des Europäischen Parlaments und des Rates zur "Förderung der Verwendung von Biokraftstoffen oder anderen erneuerbaren Kraftstoffen im Verkehrssektor" DME als Biokraftstoff gilt, darf dieser Verunreinigungen enthalten, die bei hochreinem DME nicht zugelassen sind. Es kann daher auf die Destillation des MeOH verzichtet und das Roh-MeOH direkt dem DME-Reaktor aufgegeben werden. So wird bei dem in der US 5750799 A beschriebenen Verfahren unbehandeltes Roh-MeOH direkt in einen DME-Reaktor geleitet, so dass der das MeOH enthaltende Rücklauf mit erheblichen Wassermengen belastet ist. Dieses im Kreislauf geführte Wasser muss zusätzlich zum nicht-umgesetzten MeOH kondensiert und anschließend vor dem DME-Reaktor verdampft werden, wodurch die Energieeffiziens des Verfahrens beachtlich beeinträchtigt wird. Außerdem wird durch den verminderten MeOH-Umsatz im DME-Reaktor der Kreislaufstrom vergrößert und demzufolge müssen die Apparate und apparatetechnischen Komponenten der Anlage zum Herstellen von DME größer ausgebildet werden. Roh-MeOH enthält Kohlensäure H₂CO₃ sowie geringe Mengen organischer Säuren, die neutralisiert werden müssen, um Korrosionserscheinungen an aus Stahl bestehenden Apparaten und apparatetechnischen Komponenten für das Herstellen von DME zu vermeiden. Zur Neutralisation des Roh-MeOH wird üblicherweise Soda eingesetzt.

Die US 6740783 B1 beschreibt ein Verfahren zur Herstellung von DME aus Roh-MeOH unter Verwendung eines DME-Reaktors mit einem Festbett aus ZeolithKatalysator, der zunächst durch eine Dotierung mit Metallen deaktiviert wird, um die DME-Selektivität des Katalysators zu steigern. Eine stetige Zufuhr von Metallen führt jedoch langfristig zu einer kontinuierlichen Deaktivierung und damit zu einer Verkürzung der Lebensdauer des Katalysators. Gegenstand der EP 1396483 B1 ist ein Verfahren zur Herstellung von DME, bei dem Roh-MEOH in der Dampfphase in Gegenwart eines aktivierten mit Na dotierten Al₂O₃-Katalysators dehydratisiert wird. Dabei muss auf eine begrenzte Na-Dotierung geachtet werden, um den Umsatz des Katalysators nicht zu beeinträchtigen. Das bedeutet, dass das Roh-MeOH weitgehend frei von Metall- und NH₄-Ionen sein muss. Bei dem bereitgestellten Roh-MeOH und dessen Verdampfung ist daher der Mitriss von Neutralisierungsmittel sorgfältig zu vermeiden.

In den Druckschriften CN 100366597 C, CN 1830934 A und JP 2004161673 A sind Vorrichtungen beschrieben, bei denen Roh-MeOH und Rücklauf-MeOH einer gemeinsamen Trenneinrichtung zugeführt werden. Das Rücklauf-MeOH enthält das gesamte aus dem DME-Reaktor stammende Reaktionswasser, so dass das Rücklauf-MeOH nicht dem Kopf der Destillationskolonne aufgegeben werden kann. Es ist deshalb der Einsatz eines Kopfkondensators vorgesehen, um den Wassergehalt des dem DME-Reaktor zugeführten Roh-MeOH zu senken. Dies erfordert eine erhebliche Kondensatorkapazität, die mit einer entsprechend größeren Leistung des Reboilers verbunden ist, wodurch die Energieeffiziens und die Wirtschaftlichkeit reduziert werden. Die JP 2004161672 A befasst sich mit einem Verdampfer für Roh-MeOH, der eine Teilverdampfung des Roh-MeOH erlaubt, wobei das nicht verdampfte Gemisch aus MeOH und Wasser gemeinsam mit dem nicht umgesetzten MeOH und dem Reaktionswasser aus dem DME-Reaktor in einer separaten bei niedrigem Druck arbeitenden Destillationskolonne in Prozesswasser und Rücklauf-MeOH getrennt wird. Das unterkühlte wasserarme flüssige Rücklauf-MeOH wird mit dem verdampften Roh-MeOH in Kontakt gebracht, so dass die Wasserkonzentration in dem dem DME-Reaktor zugeführten Roh-MeOH gesenkt wird. Es ist also vorgesehen, mit dem nicht verdampften Wasser des Roh-MeOH auch nicht verdampftes MeOH zur Rücklauf-MeOH-Kolonne zu leiten. Diese Maßnahme erfordert das Verdampfen und Kondensieren des MeOH in der Rücklauf-MeOH-Kolonne und nach dem Rücklauf des weitgehend wasserfreien MeOH zum Roh-MeOH-Verdampfer die erneute Verdampfung desselben MeOH. Es entsteht also ein MeOH-Kreislauf ohne Kontakt mit dem DME-Reaktor, der unnötige Energie verbraucht und die Effizienz der Schaltung reduziert.

Aus der EP 455004 A1 oder der US 5750799 A ist bekannt, das mit den nicht-kondensierbaren Gasen am Kopf der DME-Kolonne ausgetragene DME in einer Kolonne auszuwaschen und zur MeOH-Kolonne zurückzuführen. Diese Maßnahme führt zu einem DME-Gehalt in dem dem DME-Reaktor aufgegebenen Roh-MeOH, durch den der Umsatz von MeOH zu DME reduziert wird. Dies ist insbesondere relevant, wenn Roh-MeOH eingesetzt wird, und die in dem Roh-MeOH enthaltenen leichten Gase nicht vor dem DME-Reaktor sondern erst in der DME-Kolonne abgetrennt werden. Proportional zur Menge der nicht-kondensierten leichten Gase steigt die Menge des mit den Gasen ausgetragenen DME und damit die Menge an DME im Zulauf zum DME-Reaktor an.

Wenn das Roh-MeOH nur wenig gelöste Gase enthält, kann auf die Anordnung eines Gaswäschers verzichtet werden. Stattdessen kann in einem mit Kaltwasser oder einem Kältemittel betriebenen Endkühler das mit den nicht-kondensierbaren Gasen ausgetragene DME weitgehend zurückgewonnen werden.

Die der Erfindung zugrundeliegende Aufgabe besteht darin, das eingangs beschriebene Verfahren so zu gestalten, dass ein möglichst niedriger Verbrauch an Betriebsmitteln erreicht, die installierte Wärmeübertragerleistung verbessert und die Lebensdauer des Katalysators nicht beeinträchtigt werden.

Gelöst ist diese Aufgabe dadurch, dass das aus dem Reaktor abgezogene Reaktionsgemisch in einer Kolonne, nachfolgend als Gemisch-Kolonne bezeichnet, in ein überwiegend aus Wasser bestehendes Sumpfprodukt und in ein überwiegend aus DME und MeOH gebildetes Destillat getrennt wird, das Destillat in einer Kolonne, nachfolgend als DME-Kolonne bezeichnet, in ein im wesentlichen DME und über Kopf abgebbare nicht-kondensierbare Gase enthaltendes Destillat und in ein aus wasserarmem MeOH gebildetes Sumpfprodukt, das dem Kopf der MeOH-Kolonne zugeführt wird, getrennt wird.

Das Begriff "Destillat" definiert das bei einer Destillations- bzw. Rektifikationskolonne als Seitenprodukt oder als Kopfprodukt abgenommene Erzeugnis.

Im Rahmen der Ausgestaltung des Erfindung wird das aus wasserarmem MeOH gebildete Sumpfprodukt der DME-Kolonne dem Kopf der MeOH-Kolonne zugeleitet oder mit dem aus der Vorlaufkolonne abgezogenen Sumpfprodukt gemischt.

Nach einem weiteren Erfindungsmerkmal besteht die Möglichkeit, das aus dem Reaktor abgezogene Reaktionsgemisch in einem prozessinternen Wärmeübertrager abzukühlen oder teilweise zu kondensieren, bevor das Reaktionsgemisch der Gemisch-Kolonne aufgegeben wird.

Die Wassermenge des in die DME-Kolonne eingeleiteten, überwiegend aus DME und MeOH bestehenden Destillats der Gemisch-Kolonne, ist über das Rücklaufverhältnis von der am Kolonnenkopf durch Kondensation eines Teils des Kopfprodukts erzeugter und am Kolonnenkopf wieder aufgegebener Flüssigkeitsmenge und der zur DME-Kolonne abgeführten Kopfproduktmenge einstellbar. Durch diese Maßnahme lässt sich die Qualität des aus dem Sumpf der DME-Kolonne abgezogenen wasserarmen flüssigen Rücklauf-MeOH optimieren.

Ein weiteres Erfindungsmerkmal ist darin zu sehen, dass ein Teil des durch Verdampfen des Rücklaufs des im wesentlichen aus Wasser bestehenden Sumpfprodukts der MeOH-Kolonne erzeugten Dampfs in den Sumpf der Gemisch-Kolonne geleitet und auf diese Weise der DME-Gehalt im Sumpf gesenkt wird.

Eine Ausgestaltung der Erfindung ist ferner darin zu sehen, dass das im wesentlichen aus DME bestehende Destillat der DME-Kolonne kondensiert und ein Teil des Kondensats als Rücklauf dem Kopf der DME-Kolonne aufgegeben und der andere Teil des DME-Kondensats ausgeleitet wird. Dadurch, dass gleichzeitig das aus wasserarmem flüssigen MeOH bestehende Sumpfprodukt der DME-Kolonne am Kopf der MeOH-Kolonne aufgegeben wird, kann auf die Kondensation in der MeOH-Kolonne verzichtet werden.

Es besteht auch die Möglichkeit, das in der DME-Kolonne kondensierte DME als Seitenprodukt aus dem Verstärkungsteil der DME-Kolonne auszuleiten.

Ein weiteres Merkmal der Erfindung ist darin zu sehen, dass das mit den nicht-kondensierbaren Gasen ausgetragene DME in einer Waschkolonne, vorzugsweise mit MeOH, ausgewaschen und in die Gemisch-Kolonne geleitet wird.

Im Rahmen der zusätzlichen Ausgestaltung der Erfindung werden vor dem Verdampfen des MeOH in der MeOH-Kolonne von dem zulaufenden Roh-MeOH die darin gelösten Gasen und leicht-siedenden Komponenten in einer Vorlaufkolonne abgetrennt und das Destillat ausgeleitet, wodurch die Qualität des erzeugten DME verbessert und die mit den nicht-kondensierbaren Gasen aus der DME-Kolonne mitgeschleppte DME-Menge verkleinert wird.

Das aus der Vorlaufkolonne abgezogene Sumpfprodukt wird vor dem Einlauf in die MeOH-Kolonne vorgewärmt und/oder teilweise verdampft.

Eine Ausgestaltung des erfindungsgemäßen Verfahrens besteht ferner darin, dass das am Kopf der DME-Kolonne mit den nicht-kondensierbaren Gasen ausgetragene DME in einer Waschkolonne, vorzugsweise mit MeOH, ausgewaschen und das erzeugtes Sumpfprodukt in die DME-Kolonne geleitet und/oder dem Roh-MeOH vor dem Eintritt in die MeOH-Kolonne zugesetzt wird.

Nach einem weiteren Erfindungsmerkmal kann von dem Zulauf des Roh-MeOH abgezweigtes Roh-MeOH als Waschmittel am Kopf der Waschkolonne aufgegeben werden.

Wenn das Roh-Methanol nur geringe Mengen gelöste Gase enthält, kann auf die Anordnung einer Waschkolonne verzichtet werden und stattdessen ein mit Kaltwasser oder Kältemittel, beispielsweise DME, betriebener Endkühler eingesetzt werden, mit dem sich das mit den nicht kondensierbaren Gasen ausgetragene DME zurückgewinnen lässt.

Es ist zweckmäßig, das gelöste Gase und leicht-siedende Komponenten enthaltende Destillat der Vorlaufkolonne mit dem nicht-kondensierbare Gase enthaltenden Destillat der Waschkolonne zusammenzuführen und das Gemisch zur Weiterverarbeitung auszuleiten.

Nach einem zusätzlichen Erfindungsmerkmal wird das überwiegend aus Wasser bestehende Sumpfprodukt der MeOH-Kolonne aus dem Prozess ausgeleitet oder dem unteren Teil der Gemisch-Kolonne zugeführt und dann über den Sumpf der Gemisch-Kolonne ausgeleitet.

Es besteht auch die Möglichkeit, das überwiegend aus Wasser bestehende Sumpfprodukt der Gemisch-Kolonne in die MeOH-Kolonne zu leiten und das Wasser über den Sumpf der MeOH-Kolonne aus dem Prozess auszuleiten.

Es ist von Vorteil, das MeOH enthaltende Destillat der MeOH-Kolnne vor dem Einleiten in den Reaktor durch indirekte Wärmeübertragung von der in dem aus dem Reaktor ausgetragenen Reaktionsgemisch enthaltenen Reaktionswärme auf eine Reaktionstemperatur von 250 bis 330° C zu überhitzen.

Ein Teil des gasförmigen Destillats der MeOH-Kolonne wird in den Sumpf der DME-Kolonne geleitet.

Bei der Vorrichtung zur Durchführung des Verfahrens ist erfindungsgemäß die DME-Kolonne auf der MeOH-Kolonne angeordnet. Die beiden Kolonnen sind über das Destillat der MeOH-Kolonne und das Sumpfprodukt der DME-Kolonne gekoppelt, so dass die MeOH-Kolonne ohne Rücklaufkühler und die DME-Kolonne ohne Verdampfer betrieben werden können.

Eine Abwandlung des erfindungsgemäßen Verfahrens besteht darin, dass dampfförmige überwiegend aus DME und MeOH bestehende Destillat der Gemisch-Kolonne in den Sumpf einer Rektifikationskolonne, nachfolgend als DME-Produkt-Kolonne bezeichnet, eingespeist wird, das flüssiges MeOH enthaltende Sumpfprodukt dieser Kolonne am Kopf einer Rektifikationskolonne, nachfolgend als MeOH-Rücklauf-Kolonne bezeichnet, aufgegeben wird, wobei deren Destillat in den Sumpf der DME-Produkt-Kolonne geleitet und deren Sumpfprodukt dem Kopf der MeOH-Kolonne zugeführt wird.

Bei der erfindungsgemäßen Abwandlung des Verfahrens sind die als Verstärkungsteil ausgebildete DME-Produkt-Kolonne auf der Gemisch-Kolonne und die als Abtriebsteil ausgebildete MeOH-Rücklauf-Kolonne auf der MeOH-Kolonne angeordnet. Durch diese Maßnahme sind praktisch der Verstärkungsteil und der Abtriebsteil der DME-Kolonne getrennt und der kleinere Abtriebsteil der DME-Kolonne lässt sich auf die Methanol-Kolonne und der größere Verstärkungsteil der DME-Kolonne auf die Gemisch-Kolonne stellen. Der Durchmesser von Gemisch-Kolonne und Verstärkungsteil der DME-Kolonne sind nahezu gleich groß.

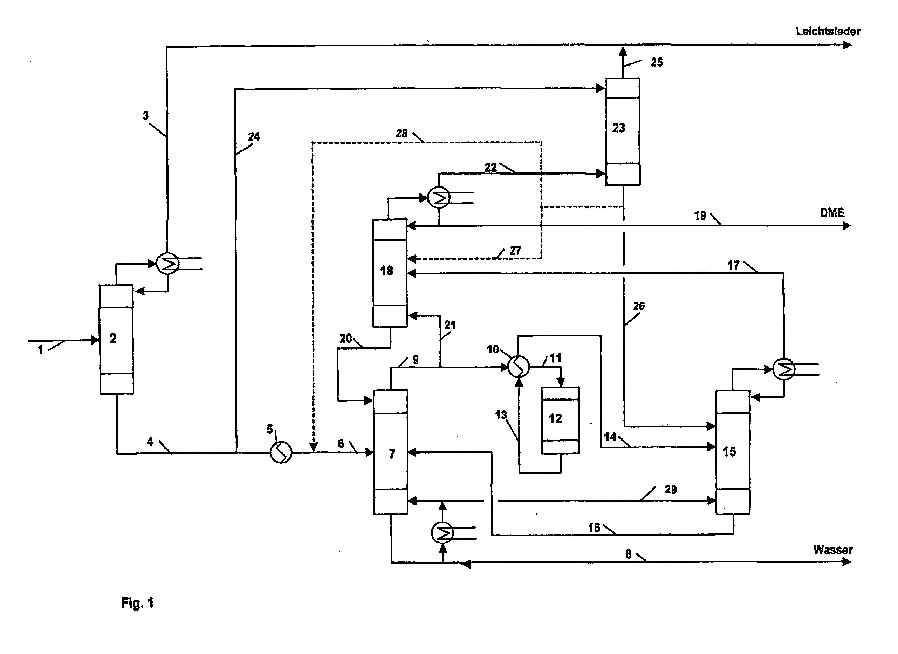

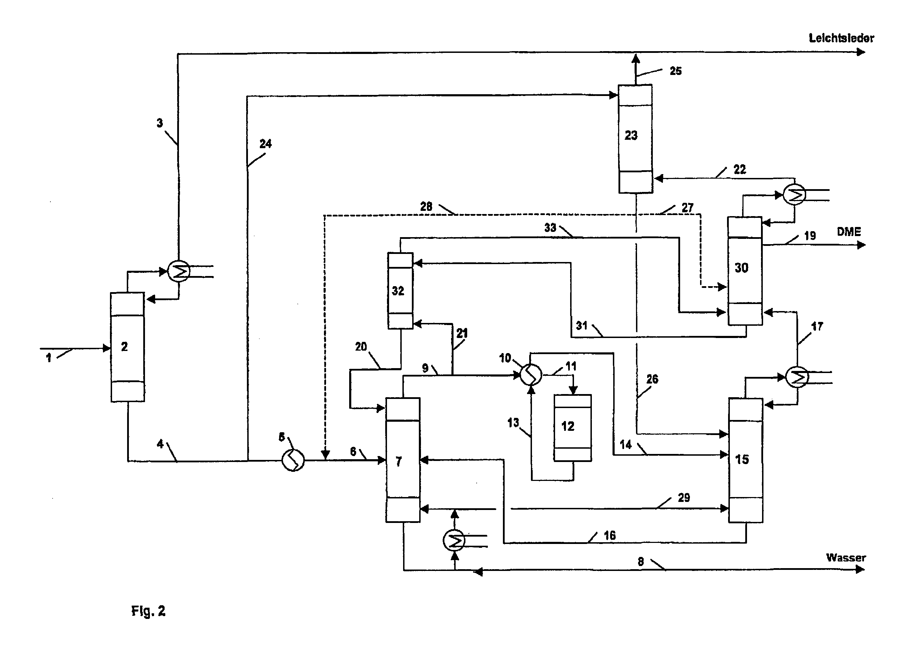

Weitere Merkmale, Vorteile und Anwendungsmöglichkeiten des erfindungsgemäßen Verfahrens ergeben sich auch aus der nachfolgenden Beschreibung eines Ausführungsbeispiels und der in der Zeichnung in Fig. 1 und Fig. 2 dargestellten apparativen Verfahrensfließbilder. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbeziehung.

Gemäß Fig. 1 wird ein aus Roh-MeOH mit einem MeOH-Gehalt von 75 Gew. % bestehender Einsatzstrom über Leitung (1) in die Vorlauf-Kolonne (2) eingespeist, in der bei einer mittleren Temperatur von 80° C und einem mittleren Druck von 3 bar[a] die in dem Roh-MeOH gelösten Gase, wie CO, CO₂, CH₄ und die leichtsiedenden Kohlenwasserstoffe und über Leitung (3) zur anderweitigen Nutzung ausgeleitet werden. Das mit einer mittleren Temperatur von 100° C vom Sumpf der Vorlaufkolonne (2) über Leitung (4) abgezogene MeOH wird in einer Wärmeübertragergruppe (5) auf eine mittlere Temperatur von 160° C vorgewärmt, dabei teilweise verdampft und über Leitung (6) einer MeOH-Kolonne (7) aufgegeben, in der bei einer mittleren Temperatur von 180° C und einem mittleren Druck von 20 bar[a] das Wasser vom MeOH abgetrennt wird. Aus dem Sumpf der MeOH-Kolonne (7) wird das Wasser über Leitung (8) aus dem Prozess ausgeleitet, während das über Leitung (9) abgeführte gasförmige eine mittlere Temperatur von 170° C besitzende Kopfprodukt der MeOH-Kolonne in einer Wärmeübertragergruppe (10) auf eine mittlere Temperatur von 300° C erwärmt und über Leitung (11) in einen Reaktor (12) geleitet wird. Das am Boden des Reaktors (12) abgezogene Gasgemisch wird über Leitung (13) der Wärmeübertragergruppe (10) zugeführt und rekuperativ gekühlt, bevor es über Leitung (14) der Gemisch-Kolonne (15) aufgegeben wird. In der Gemisch-Kolonne (15) wird das Gasgemisch bei einem mittleren Druck von 15 bar[a] und einer mittleren Temperatur von 150° C in ein wasserreiches Sumpfprodukt und in ein wasserarmes Kopfprodukt getrennt, wobei das Kopfprodukt weniger als 5 Gew. %, vorzugsweise weniger als 2 Gew. %, Wasser enthält. Das wasserreiche Sumpfprodukt wird über Leitung (16) zur MeOH-Kolonne (7) geleitet. Das wasserarme Kopfprodukt wird über Leitung (17) der DME-Kolonne (18), die bei einer mittleren Temperatur von 100° C und einem Druck von 13,5 bar[a] arbeitet, zugeführt. Vom Kopf der DME-Kolonne (18) oder von der Seite, zwei bis sieben Böden unterhalb des Kopfkondensators, wird DME abgezogen und über Leitung (19) zur Anlagengrenze geleitet. Das überwiegend aus MeOH mit kleinen Mengen an Wasser bestehende Sumpfprodukt der DME-Kolonne (18) wird über Leitung (20) zum Kopf der MeOH-Kolonne (7) gepumpt und dient als Rückfluss für den Verstärkungsteil der MeOH-Kolonne (7). Um die Konzentration von DME im Sumpf der DME-Kolonne (18) möglichst klein zu halten, kann ein Teilstrom des in Leitung (9) strömenden Kopfprodukts der MeOH-Kolonne (7) abgezweigt und über Leitung (21) in den Sumpf der DME-Kolonne (18) eingeleitet werden, so dass in diesem Fall auf einen Verdampferkreislauf verzichtet werden kann.

Die in der DME-Kolonne (18) nicht-kondensierbaren Gase werden über Kopf abgezogen und über Leitung (22) in den Sumpf der Waschstufe (23) geleitet, in der bei einem mittleren Druck von 10 bar[a] und einer mittleren Temperatur von 75° C das in den nicht-kondensierbaren Gasen enthaltene DME mit aus der Leitung (4) abgezweigtem und über Leitung (24) am Kopf der Waschstufe (23) aufgegebenem MeOH zurückgewonnen wird. Das gasförmige Kopfprodukt der Waschstufe (23) wird über Leitung (25) mit dem über Leitung (3) abströmenden Kopfprodukt der Vorlaufkolonne (2) zusammengeführt und zur anderweitigen Benutzung ausgeleitet. Das DME enthaltende Waschmittel wird aus dem Sumpf der Waschstufe (23) über Leitung (26) in die Gemisch-Kolonne (15) gepumpt. Alternativ kann das Sumpfprodukt der Waschstufe (23) über Leitung (27) entweder in die DME-Kolonne (18) oder über Leitung (28) in den in Leitung (6) der MeOH-Kolonne (7) zuströmenden MeOH-Strom eingespeist werden. Die Gemisch-Kolonne (15) kann ohne Verdampferkreislauf betrieben werden.

Bei der in Fig. 2 wiedergegebenen Abwandlung des Verfahrensfließbilds nach Fig. 1 wird das über Leitung (17) vom Kopf der Gemisch-Kolonne (15) abgeführte wasserarme Produkt unmittelbar in den Sumpf einer als Verstärkungskolonne ausgebildeten auf der Gemischkolonne (15) angebrachten Rektifikationskolonne (30), nachfolgend als DME-Produkt-Kolonne bezeichnet, geleitet. Das überwiegend aus MeOH mit kleinen Mengen an Wasser bestehende Sumpfprodukt der DME-Produkt-Kolonne (30) wird über Leitung (31) dem Kopf einer auf der MeOH-Kolonne (7) angeordneten als Abtriebskolonne ausgebildeten Kolonne (32), nachfolgend als MeOH-Rücklauf-Kolonne bezeichnet, zugeführt. Das Sumpfprodukt der MeOH-Rücklauf-Kolonne (32) strömt über Leitung (20) zum Kopf der MeOH-Kolonne (7), während das Kopfprodukt über Leitung (33) in den Sumpf der DME-Produkt-Kolonne (30) geleitet wird. Der von dem in Leitung (9) fließenden Kopfprodukt der MeOH-Kolonne (9) abgezweigte Teilstrom wird über Leitung (21) dem Sumpf der MeOH-Rücklauf-Kolonne (32) zugeführt.

Die mit der Erfindung erzielten Vorteile sind insbesondere in einer vergleichsweise verbesserten Energieeffizienz und Wirtschaftlichkeit zu sehen. Ein Vergleich des erfindungsgemäßen Verfahrens mit einem bekannten zum Stand der Technik gehörenden Verfahren, wie dieses beispielsweise in der JP 2004161672 A beschrieben ist, zeigt, dass bei gleichen Randbedingungen für den MeOH-Gehalt des Roh-MeOH, die Vorwärmung und Teilverdampfung des MeOH vor Eintritt in die MeOH-Kolonne, die Eintrittstemperatur in den Reaktor, die Reinheit des erzeugten DME und die Reinheit des gewonnen Prozesswassers und bei nahezu gleichem externen Energieverbrauch die total installierte Wärmetauscherleistung um etwa 20 % kleiner als bei dem bekannten Verfahren ist.

## Patentansprüche

1. Verfahren zum Herstellen von Dimethylether (DME) aus Methanol (MeOH), durch Umsetzen, vorzugsweise durch säurenkatalytische Kondensation, von durch MeOH-Synthese gewonnenem Roh-MeOH zu DME unter Abspalten von Wasser in einem Reaktor (12), bei dem das aus Roh-MeOH bestehende Einsatzgemisch und wenigstens ein prozessintern gewonnener im wesentlichen aus nicht umgesetztem MeOH und Reaktionswasser gebildeter Rückfluss einer MeOH-Kolonne (7) aufgegeben und verdampft werden und das im wesentlichen aus dampfförmigem MeOH bestehende Destillat dem Reaktor zugeführt wird, **dadurch gekennzeichnet, dass** das aus dem Reaktor (12) abgezogene Reaktionsgemisch in einer Gemisch-Kolonne (15) in ein überwiegend aus Wasser bestehendes Sumpfprodukt und in ein überwiegend aus DME und MeOH gebildetes Destillat getrennt wird, das Destillat in einer DME-Kolonne (18) in ein im wesentlichen DME und über Kopf abgegebene nicht-kondensierbare Gase enthaltendes Destillat und in ein aus wasserarmem MeOH gebildetes Sumpfprodukt, das dem Kopf der MeOH-Kolonne (7) zugeführt oder mit dem aus der Vorlauf-Kolonne (2) abgezogenen Sumpfprodukt gemischt wird, getrennt wird.

2. Verfahren nach Anspruch 1, **dadurch gekennzeichnet, dass** das aus dem Reaktor (12) abgezogene Reaktionsgemisch in einem prozessinternen Wärmeübertrager (10) abgekühlt oder teilkondensiert wird, bevor es Gemisch-Kolonne (15) aufgegeben wird.

3. Verfahren nach einem der Ansprüche 1 und 2, **dadurch gekennzeichnet, dass** die Wassermenge des der DME-Kolonne (18) zugeführten überwiegend aus DME und MeOH bestehenden Destillats der Gemisch-Kolonne (15) über das Rücklaufverhältnis von der am Kolonnenkopf durch Kondensation eines Teils des Kopfprodukts erzeugter am Kolonnenkopf wieder aufgegebener Flüssigkeitsmenge und der zur DME-Kolonne abgeführten Kopfproduktmenge einstellbar ist.

4. Verfahren nach einem der Ansprüche 1 bis 3, **dadurch gekennzeichnet, dass** ein Teil des durch Verdampfen des Rücklaufs des im wesentlichen aus Wasser bestehenden Sumpfprodukts der MeOH-Kolonne (7) erzeugten Dampfs in den Sumpf der Gemisch-Kolonne (15) geleitet wird.

5. Verfahren nach einem der Ansprüche 1 bis 4, **dadurch gekennzeichnet, dass** das im wesentlichen aus DME bestehende Destillat der DME-Kolonne (18) kondensiert und ein Teil des Kondensats als Rücklauf dem Kopf der DME-Kolonne aufgegeben und der andere Teil ausgeleitet wird.

6. Verfahren nach einem der Ansprüche 1 bis 5, **dadurch gekennzeichnet, dass** das in der DME-Kolonne (18) kondensierte DME als Seitenprodukt aus dem Auftriebsteil der DME-Kolonne ausgeleitet wird.

7. Verfahren nach einem der Ansprüche 1 bis 6, **dadurch gekennzeichnet, dass** das mit den nicht-kondensierbaren Gasen aus der DME-Kolonne (18) ausgetragene DME in einer Waschkolonne (23), vorzugsweise mit MeOH, ausgewaschen und in die Gemisch-Kolonne (15) geleitet wird.

8. Verfahren nach einem der Ansprüche 1 bis 7, **dadurch gekennzeichnet, dass** von dem Zulauf an zu trennendem Roh-MeOH vor dem Verdampfen des MeOH in der MeOH-Kolonne (7) die in dem Roh-MeOH gelösten Gase und leicht-siedenden Komponenten in einer Vorlaufkolonne (2) abgetrennt und das Destillat aus dem Prozess ausgeleitet wird.

9. Verfahren nach einem der Ansprüche 1 bis 8, **dadurch gekennzeichnet, dass** der Zulauf an zu trennendem Roh-MeOH vor der MeOH-Kolonne (7) vorgewärmt und/oder teilweise verdampft wird.

10. Verfahren nach einem der Ansprüche 1 bis 9, **dadurch gekennzeichnet, dass** das mit nicht-kondensierbaren Gasen aus der DME-Kolonne (18) ausgetragene DME in einer Waschkolonne (23), vorzugsweise mit MeOH, ausgewaschen und das erzeugte Sumpfprodukt in die DME-Kolonne (18) geleitet und/oder dem Roh-MeOH vor dem Eintritt in die MeOH-Kolonne (7) zugesetzt wird.

11. Verfahren nach einem der Ansprüche 1 bis 10, **dadurch gekennzeichnet, dass** als Waschmittel von dem Zulauf an MeOH abgezweigtes Roh-MeOH am Kopf der Waschkolonne(23) aufgegeben wird oder ein Teilstrom des Sumpfprodukts der DME-Kolonne (18) eingesetzt wird.

12. Verfahren nach einem der Ansprüche 1 bis 11, **dadurch gekennzeichnet, dass** das gelöste Gase und leicht-siedende Komponenten enthaltende Destillat der Vorlaufkolonne (2) mit dem nicht-kondensierbare Gase enthalten Kopfprodukt der Waschkolonne (23) zusammengeführt wird.

13. Verfahren nach einem der Ansprüche 1 bis 12, **dadurch gekennzeichnet, dass** das überwiegend aus Wasser bestehende Sumpfprodukt der MeOH-Kolonne (7) in den unteren Teil der Gemisch-Kolonne (15) geleitet oder aus dem Prozess geleitet wird.

14. Verfahren nach einem der Ansprüche 1 bis 13, **dadurch gekennzeichnet, dass** das übverwiegend aus Wasser bestehende Sumpfprodukt der Gemisch-Kolonne (15) in die MeOH-Kolonne (7) geleitet oder aus dem Prozess geleitet wird.

15. Verfahren nach einem der Ansprüche 1 und 14, **dadurch gekennzeichnet, dass** das MeOH enthaltende Destillat der MeOH-Kolonne (7) vor dem Einleiten in den Reaktor (12) durch indirekte Wärmeübertragung von der in dem aus dem Reaktor ausgetragenen Reaktionsgemisch enthaltenen Reaktionswärme auf eine Reaktionstemperatur von 250 bis 330° C überhitzt wird.

16. Verfahren nach einem der Ansprüche 1 bis 15, **dadurch gekennzeichnet, dass** ein Teil des Destillats der MeOH-Kolonne (7) in den Sumpf der DME-Kolonne (18) geleitet wird.

17. Abwandlung des Verfahrens nach einem der Ansprüche 1 bis 16, **dadurch gekennzeichnet, dass** das dampfförmige überwiegend aus DME und MeOH bestehende Destillat der Gemisch-Kolonne (15) in den Sumpf einer DME-Produkt-Kolonne (30) eingespeist wird, das flüssiges MeOH enthaltende Sumpfprodukt der DME-Produkt-Kolonne am Kopf einer MeOH-Rücklauf-Kolonne (32) aufgegeben wird, deren Destillat in den Sumpf der DME-Produkt-Kolonne (30) geleitet und deren Sumpfprodukt am Kopf der MeOH-Kolonne (7) aufgegeben oder dem aus der Vorlauf-Kolonne (2) abgezogenen Sumpfprodukt zugesetzt wird.

## Claims

1. A process for producing dimethyl ether (DME) from methanol (MeOH) by converting, preferably by acid-catalytic condensation, raw MeOH obtained through MeOH synthesis to DME by splitting off water in a reactor (12), in which the feed mixture consisting of raw MeOH and a process-internally obtained return flow substantially formed from unconverted MeOH and reaction water, are charged to a MeOH column (7) and evaporated, and the distillate substantially consisting of vaporous MeOH is supplied to the reactor, **characterized in that** in a mixture column (15) the reaction mixture withdrawn from the reactor (12) is separated into a bottom product chiefly consisting of water and into a distillate chiefly formed from DME and MeOH, in a DME column (18) the distillate is separated into a distillate substantially containing DME and uncondensable gases discharged overhead and into a bottom product formed from water-poor MeOH, which is supplied to the top of the MeOH column (7) or mixed with the bottom product withdrawn from the forerun column (2).

2. The process according to claim 1, **characterized in that** the reaction mixture withdrawn from the reactor (12) is cooled in a process-internal heat exchanger (10) or partially condensed, before it is charged to the mixture column (15).

3. The process according to any of claims 1 and 2, **characterized in that** the amount of water of the distillate of the mixture column (15) supplied to the DME column (18), which chiefly consists of DME and MeOH, is adjustable via the reflux ratio of the amount of liquid produced at the top of the column by condensation of a part of the top product and charged again to the top of the column and the amount of top product discharged to the DME column.

4. The process according to any of claims 1 to 3, **characterized in that** a part of the vapor produced by evaporating the return flow of the bottom product of the MeOH column (7), which substantially consists of water, is passed into the bottom of the mixture column (15).

5. The process according to any of claims 1 to 4, **characterized in that** the distillate of the DME column (18) substantially consisting of DME is condensed and one part of the condensate is charged to the top of the DME column as return flow and the other part is discharged.

6. The process according to any of claims 1 to 5, **characterized in that** the DME condensed in the DME column (18) is discharged from the ascending section of the DME column as side product.

7. The process according to any of claims 1 to 6, **characterized in that** the DME discharged from the DME column (18) with the uncondensable gases is washed out in a washing column (23), preferably with MeOH, and passed into the mixture column (15).

8. The process according to any of claims 1 to 7, **characterized in that** before evaporation of the MeOH in the MeOH column (7) the gases and low-boiling components dissolved in the raw MeOH are separated from the inflow of raw MeOH to be separated in a forerun column (2), and the distillate is discharged from the process.

9. The process according to any of claims 1 to 8, **characterized in that** the inflow of raw MeOH to be separated is preheated and/or partly evaporated before the MeOH column (7).

10. The process according to any of claims 1 to 9, **characterized in that** the DME discharged from the DME column (18) with the uncondensable gases is washed out in a washing column (23), preferably with MeOH, and the bottom product produced is passed into the DME column (18) and/or added to the raw MeOH prior to entry into the MeOH column (7).

11. The process according to any of claims 1 to 10, **characterized in that** raw MeOH branched off from the inflow of MeOH as washing agent is charged at the top of the washing column (23) or a partial stream of the bottom product of the DME column (18) is used.

12. The process according to any of claims 1 to 11, **characterized in that** the distillate of the forerun column (2), which contains dissolved gases and low-boiling components, is combined with the top product of the washing column (23) which contains uncondensable gases.

13. The process according to any of claims 1 to 12, **characterized in that** the bottom product of the MeOH column (7), which chiefly consists of water, is passed into the lower part of the mixture column or discharged from the process.

14. The process according to any of claims 1 to 13, **characterized in that** the bottom product of the mixture column (15), which chiefly consists of water, is passed into the MeOH column (7) or discharged from the process.

15. The process according to any of claims 1 and 14, **characterized in that** before introduction into the reactor (12) the distillate of the MeOH column (7), which contains MeOH, is superheated to a reaction temperature of 250 to 330°C by indirect heat transfer from the reaction heat contained in the reaction mixture discharged from the reactor.

16. The process according to any of claims 1 to 15, **characterized in that** a part of the distillate of the MeOH column (7) is passed into the bottom of the DME column (18).

17. Modification of the process according to any of claims 1 to 16, **characterized in that** the vaporous distillate of the mixture column (15), which chiefly consists of DME and MeOH, is fed into the bottom of a DME product column (30), the bottom product of the DME product column, which contains liquid MeOH, is charged to the top of an MeOH reflux column (32), whose distillate is passed into the bottom of the DME product column (30) and whose bottom product is charged to the top of the MeOH column (7) or added to the bottom product withdrawn from the forerun column (2).

## Revendications

1. Procédé de préparation d'oxyde diméthylique (DME) à partir de méthanol (MeOH), par réaction, de préférence par condensation à catalyse acide, de MeOH brut obtenu par synthèse de MeOH en du DME avec élimination d'eau dans un réacteur (12), dans lequel on charge et on évapore dans une colonne (7) de MeOH le mélange de charge constitué de MeOH brut et d'au moins un reflux obtenu d'une manière interne à l'opération et formé essentiellement de MeOH n'ayant pas réagi et d'eau de réaction et on envoie au réacteur le distillat constitué essentiellement de MeOH sous forme de vapeur, **caractérisé en ce que** l'on sépare le mélange de réaction soutiré du réacteur (12) dans une colonne (15) de mélange en un produit de pied constitué d'une manière prépondérante d'eau et en un distillat formé d'une manière prépondérante de DME et de MeOH, on sépare le distillat dans une colonne (18) de DME en un distillat, contenant essentiellement du DME et des gaz non condensables éliminés en tête, et en un produit de pied formé de MeOH pauvre en eau, que l'on envoie en tête de la colonne (7) de MeOH ou que l'on mélange au produit de pied soutiré de la colonne (2) primaire.

2. Procédé suivant la revendication 1, **caractérisé en ce que** l'on refroidit ou **en ce que** l'on condense partiellement, dans un échangeur de chaleur (10) interne à l'opération, le mélange de réaction soutiré du réacteur (12), avant de le charger dans la colonne (15) de mélange.

3. Procédé suivant l'une des revendications 1 et 2, **caractérisé en ce que** la quantité d'eau du distillat de la colonne (15) de mélange, constitué d'une manière prépondérante de DME et de MeOH envoyée à la colonne (18) de DME, est réglable par le rapport de reflux de la quantité de liquide, produite en tête de colonne par condensation d'une partie du produit de tête et rechargée en tête de colonne, et de la quantité du produit de tête évacuée vers la colonne de DME.

4. Procédé suivant l'une des revendications 1 à 3, **caractérisé en ce que** l'on envoie au pied de la colonne (15) de mélange une partie de la vapeur produite par évaporation du reflux du produit de pied de la colonne (7) de MeOH, constituée essentiellement d'eau.

5. Procédé suivant l'une des revendications 1 à 4, **caractérisé en ce que** l'on condense le distillat de la colonne (18) de DME constitué essentiellement de DME et l'on charge une partie du produit condensé comme reflux en tête de la colonne de DME et l'on fait sortir l'autre partie.

6. Procédé suivant l'une des revendications 1 à 5, **caractérisé en ce que** l'on écarte de la partie haute de la colonne de DME, en tant que fraction latérale, le DME condensé dans la colonne (18) de DME.

7. Procédé suivant l'une des revendications 1 à 6, **caractérisé en ce que** l'on lave, de préférence par du MeOH, dans une colonne (23) de lavage, le DME extrait avec les gaz non condensables de la colonne (18) de DME et on l'envoie dans la colonne (15) de mélange.

8. Procédé suivant l'une des revendications 1 à 7, **caractérisé en ce que**, de la charge de MeOH brut à séparer, on sépare, dans une colonne (2) primaire, avant l'évaporation du MeOH dans la colonne (7) de MeOH, les gaz dissous dans le MeOH brut et les constituants bouillant facilement et on écarte le distillat de l'opération.

9. Procédé suivant l'une des revendications 1 à 8, **caractérisé en ce que**, avant la colonne (7) de MeOH, on chauffe au préalable et/ou on évapore partiellement la charge de MeOH brut à séparer.

10. Procédé suivant l'une des revendications 1 à 9, **caractérisé en ce que** l'on lave, de préférence par du MeOH, dans une colonne (23) de lavage, le DME extrait de la colonne (18) de DME avec des gaz non condensables et **en ce que** l'on envoie le produit de pied produit dans la colonne (18) de DME et/ou on l'ajoute au MeOH brut avant l'entrée dans la colonne (7) de MeOH.

11. Procédé suivant l'une des revendications 1 à 10, **caractérisé en ce que** l'on charge en tête de la colonne (23) de lavage, comme agent de lavage, du MeOH brut dérivé de la charge de MeOH ou on utilise un courant partiel du produit de pied de la colonne (18) de DME.

12. Procédé suivant l'une des revendications 1 à 11, **caractérisé en ce que** l'on réunit le distillat, contenant des gaz dissous et des constituants bouillant facilement de la colonne (2) primaire, au produit de tête de la colonne (23) de lavage contenant des gaz non condensables.

13. Procédé suivant l'une des revendications 1 à 12, **caractérisé en ce que** l'on envoie, dans la partie inférieure de la colonne (15) de mélange, le produit de pied de la colonne (7) de MeOH constitué d'une manière prépondérante d'eau ou on l'écarte de l'opération.

14. Procédé suivant l'une des revendications 1 à 13, **caractérisé en ce que** l'on envoie dans la colonne (7) de MeOH le produit de pied de la colonne (15) de mélange constitué d'une manière prépondérante d'eau ou on l'écarte de l'opération.

15. Procédé suivant l'une des revendications 1 à 14, **caractérisé en ce que** l'on surchauffe à une température de réaction de 250 à 330°C le distillat de la colonne (7) de MeOH contenant du MeOH, avant l'envoi dans le réacteur (12), par transmission de chaleur indirecte de la chaleur de réaction contenue dans le mélange de réaction extrait du réacteur.

16. Procédé suivant l'une des revendications 1 à 15, **caractérisé en ce que** l'on envoie une partie du distillat de la colonne (7) de MeOH dans le pied de la colonne (18) de DME.

17. Variante du procédé suivant l'une des revendications 1 à 16, **caractérisée en ce que** l'on envoie le distillat de la colonne (15) de mélange sous forme de vapeur, constitué d'une manière prépondérante de DME et de MeOH, dans le pied d'une colonne (30) de DME produit, on charge le produit de pied de la colonne de DME produit contenant le MeOH liquide en tête d'une colonne (32) de reflux de MeOH, dont on envoie le distillat dans le pied de la colonne (30) de DME produit et dont on charge le produit de pied en tête de la colonne (7) de MeOH ou on l'ajoute au produit de pied soutiré de la colonne (2) primaire.
